# EUROPEAN PATENT SPECIFICATION

(11) **EP 1 494 752 B1**
(45) Date of publication and mention of the grant of the patent: **30.07.2008**
(21) Application number: 03719469.3
(22) Date of filing: 11.04.2003
(51) Int. Cl.: A61N 1/05

(54) **METHOD OF TREATING BIOLOGICAL MATERIALS WITH TRANSLATING ELECTRICAL FIELDS AND ELECTRODE POLARITY REVERSAL**
VERFAHREN ZUR BEHANDLUNG VON BIOLOGISCHEN MATERIALIEN MIT ÜBERSETZENDEN ELEKTRISCHEN FELDERN UND ELEKTRODEN-POLARITÄTS-UMKEHR
PROCEDE DE TRAITEMENT DE MATIERES BIOLOGIQUES AVEC DES CHAMPS ELECTRIQUES DE TRANSLATION ET UNE INVERSION DE LA POLARITE D'ELECTRODE

(30) Priority: 16.04.2002 US 372436 P
(43) Date of publication of application: 12.01.2005
(73) Proprietor: Cyto Pulse Sciences, Inc., Hanover, MD 21076 (US)
(72) Inventor: WALTERS, Richard, E., Columbia, MD 21045 (US); KING, Alan, D., Takoma Park, MD 20912 (US); DEBRUIN, Katherine, A., Durham, NC 27713 (US)
(74) Representative: Hanson, William Bennett
(86) International application number: PCT/US2003/009208
(87) International publication number: WO 2003/089046

(56) References cited:
- US-A- 6 038 480
- US-B1- 6 208 893
- US-B1- 6 241 701
- US-B1- 6 428 504

## Description

### Cross-Reference to Related Applications

This application is related to pending U. S. provisional patent application for SYSTEM FOR TREATING BIOLOGICAL MATERIALS WITH TRANSLATING ELECTRICAL FIELDS AND ELECTRODE POLARITY REVERSAL, Serial Number 60/372,436, Filing Date 16 April 2002. This application is also related to pending U. S. patent application for ELECTRODES COATED WITH TREATING AGENT AND USES THEREOF of King and Walters, Serial Number 09/920,861, Filing Date 03 August 2001, which is related to copending PCT International Application Number PCT/US00/00014, filed 12 January 2000, which is based upon copending United States Provisional Application Serial No. 60/117,755, filed 28 January 1999, and which was published on 3 August 2000 with PCT International Publication Number WO 00/44438.

### Technical Field

This invention relates to the system for delivering therapeutic materials into living cells using pulsed electric fields. More specifically, the present invention provides apparatus for delivering substances, such as macromolecules and chemotherapeutic agents into cells, in vivo, ex vivo, in vitro, and in tissues.

### Background Art

Electroporation is the reversible destabilization of cell membranes by application of a brief electric field across the cell resulting in a potential across the cell membrane. Properly administered, the destabilization results in a temporary pore or pathway through which therapeutic material can pass. The uses of electroporation are many. Some are: (1) transient introduction of DNA or RNA, (2) permanent transfection of DNA, (3) introduction of antibodies, or other proteins or drugs into cells, (4) gene therapy, and (5) cancer vaccinations, etc..

To deliver the therapeutic compounds into living cells using electroporation, a system consisting of three components is required: (1) a pulse voltage waveform generator, (2) a switching device to connect the anode or cathode of the pulse voltage waveform generator to the electrodes, and (3) an electrode array to convert the pulse voltage into a pulsed electric field. The electrode can designed for in vitro delivery in an aqueous solution or for in vivo delivery into tissue.

In the most elementary system, where the array of electrodes consists of a single anode and a single cathode, the switching device is not required. The primary objective of the electrode array is to provide a uniform electric field over the area of cell treatment.

A number of patent, published applications, and literature references are relevant to these matters, and they include the following:
U. S. patents 5,674,267, issued Oct. 7, 1997, of Mir et al.
5,702,359, issued Dec. 30, 1997, of Hofmann
5,873,849, issued Feb. 23', 1999, of Bernard
5,993,434, issued Nov. 30, 1999, of Dev et al.
6,010,613, issued Jan. 4, 2000, of Walters et al.
6,014,584, issued Jan. 11, 2000, of Hofmann et al.
6,055,453, issued Apr. 25, 2000, of Hofmann et al.
6,110,161, issued Aug. 29,2000, of Mathiesen et al.
6,117,660, issued Sep. 12, 2000, of Walters et al.

### International Patent Publications

PCT/GB01/00899, published 02 March 2001, of Shirkhanzadeh
PCT/US00/00014, published 12 January 2000, of King et al.

### Literature Publications

Hofmann et al., "Electrochemotherapy: Transition from Laboratory to the Clinic", IEEE Engineering in Medicine and Biology., November/December 1996.

Mir et al., "High-efficiency gene transfer into skeletal muscle mediated by electric pulses", Proc. National Academy of Sciences, USA, Vol. 96, pp 4262-4267, April 1999.

Gehl, et al., "In vivo electroporation of skeletal muscle: threshold, efficacy and relation to electric field distribution", Biochimica et Biophysica Acta 1428 (1999) 233-240.

Loomis-Husselbee, J. W., Cullen, P. J., Irvine, R. F., & Dawson, A. P. (1991). Electroporation can cause artefacts due to solubilization of cations from the electrode plates. Aluminum ions enhance conversion of inositol 1,3,4,5-tetrakisphosphate into inositol 1,4,5-trisphosphate in electroporated L1210 cells. Biochem.J, 277, 883-885

Friedrich, U., Stachowicz, N., Simm, A., Fuhr, G., Lucas, K., Zimmermann, U. High efficiency electrotransfection with aluminum electrodes using microsecond pulses

Stapulionis, R. (1999). Electric pulse induced precipitation of biological macromolecules in electroporation. Bioelectrochem.Bioenerg., 48, 249-254

Tomov, T. & Tsoneva, I. (2000), Bioelectrochemistry., 51, 207-209.

Kotnik, T., Miklavcic, D., Mir, L.M. Cell membrane electropermeabilization by symmetrical bipolar rectangular pulses, Part II. Reduced electrolytic contamination Bockris, J.O., Reddy, A. K.N. editors, Modern electrochemistry. Plenum/Rosetta, 1977

In Mir et al (5,674,267), an array of concentric needles is suggested. In this array two needles, one anode and one cathode are selected from all of the needles of the array. A switching device is used to select many pairs to cover the treatment area. One pair of needles has coverage of only a limited area. Moreover, even by selecting all needle pairs in the area, it is not possible to provide uniform coverage of the total treatment area. In this respect, it would be desirable to provide an electrode array and a system for selecting electrodes in the electrode array that would provide uniform coverage of a total treatment area.

Hofmann (5,702,359) and Dev (5,993,434) disclose similar systems wherein two anodes and two opposing cathodes are selected at a time. That is two pairs of opposing anodes and cathodes are selected at a time. There are a total of six pairs of electrodes, of which two pairs at a time would be connected to the pulse generator by a switching device. More recently, Hofmann et al (6,014,584 and 6,055,453) disclose the use of an array of needle electrodes and connecting two opposing pairs of electrodes at a time, and rotating those pairs 90 degrees.

Bernard (5,873,849) discloses an electrode system consisting of rows of offset needle electrodes in which at least three electrodes are arrayed in an equilateral triangle, and these electrodes are connected to the pulse generator. This array consists of one or more equilateral triangles with two electrodes connected to one polarity of the pulse generator and the third electrode connected to the opposite polarity. In this system, various electrodes could be connected to treat the coverage area. The combination of one electrode with one polarity and two electrodes of the opposite polarity leave significant areas for treatment where the electric field is not effective. In this system, two electric field vectors point in different directions.

In 1999, Gehl et al. published the above-mentioned paper in which they disclose an array of two parallel rows of needle electrodes. This electrode array provides a very uniform electric field within the rows, and the electric field provided is almost as uniform as an electric field provided between two parallel plates. However, as the treatment area increases, the distance between the rows of needles increases. As the distance between the rows of needles increases, the uniformity of the field decreases, and the voltage to maintain the same electric field strength must increase.

The pulse waveforms of the switching systems in the patents and publications described thus far above were generally rectangular waves, having the same pulse width and interval, and employing a few pulses.

Then, Walters et al, in U. S. Patent No. 6,010,613, which is incorporated herein by reference, introduced waveforms, whose pulse parameters can be changed on a pulse-to-pulse basis. These waveforms are used to form pores using short duration electric field pulses (in the microsecond range) and then to move large charged molecules into the cells with a series of lower and longer duration (in the milliseconds range) electric field pulses.

Mir et al. then published, in Proc. National Academy of Science, USA, April 1999 cited above, disclosing that electric field duration of 10's of milliseconds was optimum for the transfection of skeletal muscle. These longer pulse widths produced a problem -- that of electrochemistry effects in the vicinity of the electrodes.

Shirkhanzadeh, in the PCT/GB01/00899 publication mentioned above, addressed the electrode electrochemistry effects resulting from the longer pulses by using palladium electrodes one of which was infused with hydrogen.

Using bipolar pulses can also minimize electrochemistry at electrodes. Mathiesen (6,110,161) discloses using bipolar pulses of low electric field strength and moderate duration (50 to 5000 microseconds) for in vivo electroporation of skeletal muscle, but did not specifically address the electrochemistry effects.

In one respect, using bipolar pulses may address the electrochemistry problems, but, in another respect, the use of bipolar pulses is counter productive. The first pulse will move large charged molecules in one direction and a second pulse immediately following of opposite polarity then moves the large charged molecules back. A system is needed to keep moving the large charged molecule in the same direction to improve delivery into living cells while simultaneously minimizing the electrochemistry effects.

In systems involving electroporation of cells described above, studies have been made relating to the local environments at the electrodes and on the surfaces, of the electrodes. In this respect, studies have revealed that metal ions are released by such electrodes.

In Loomis-Husselbee, J. W., Cullen, P. J., Irvine, R. F., & Dawson, A. P. (1991). Electroporation can cause artefacts due to solubilization of cations from the electrode plates. Aluminum ions enhance conversion of inositol 1,3,4,5-tetrakisphosphate into inositol 1,4,5-trisphosphate in electroporated L1210 cells. Biochem. J, 277, 883-885, Loomis-Husselbee et al demonstrated that aluminum ions are generated by aluminum electrodes during electroporation. The aluminum ions inhibited the biochemical process under investigation. The authors concluded that aluminum ions produced during electroporation can be detrimental to cells.

In Friedrich, U., Stachowicz, N., Simm, A., Fuhr, G., Lucas, K., Zimmermann, U. High efficiency electrotransfection with aluminum electrodes using microsecond pulses, Friedrich et al showed that substantial amounts of aluminum were released from aluminum electrodes during long pulses. The principle cause of the aluminum ion release was a change of pH at the electrode interface produced by electrolysis of water. Aluminum ion release was reduced when short pulses were used.

In Stapulionis, R. (1999). Electric pulse-induced precipitation of biological macromolecules in electroporation. Bioelectrochem.Bioenerg., 48, 249-254, Stapulionis et al showed that aluminum, iron, or copper ions were produced by the anode of metal electrodes during electroporation. The metal ions produced by this process precipitated macromolecules. The macromolecule precipitation resulted in reduced reagent and reduced delivery of macromolecules into cells.

In Tomov, T. & Tsoneva, I. (2000), Bioelectrochemistry., 51, 207-209, Tomov et al observed that metal ions are produced by stainless steel electrodes during electroporation similar to the release of aluminum ions from aluminum containing electrodes. More iron was released by higher electric fields, wider pulse widths and increased salt concentrations The potential for harmful effects of iron were discussed. Quantitatively less iron is released from stainless steel electrodes than is released from aluminum electrodes (shown by others).

In Kotnik, T., Miklavcic, D., Mir, L.M. Cell membrane electropermeabilization by symmetrical bipolar rectangular pulses, Part II. Reduced electrolytic contamination, Kotnik et al compared the release of aluminum from aluminum electrodes and iron from stainless steel electrodes. The effects of unipolar and bipolar pulses on metal ion release induced by the two types of metal electrodes were compared. As was seen by others, significant amounts of metal ions were released when unipolar pulses were used for electroporation. There was a significant reduction in metal ion production when bipolar pulses were used.

There is a general discussion of events occurring at interfaces of metal conductors and ionic conductors during electroporation in Bockris, J.O., Reddy, A. K.N. editors, Modern electrochemistry. Plenum/Rosetta, 1977. This discussion reveals that electrodes used for in vivo or in vitro electroporation are electronic conductors. The tissue or fluid surrounding the electrode is an ionic conductor. The interface between the two is complicated. Electrolysis occurs at the electrodes. At rest, there are ion clouds in the ionic conductor at the interface that induce a charge equal in strength and opposite in charge within the electronic conductor. When an electrical potential is applied across at least two oppositely charged electrodes (the electronic conductors) a current is induced across the ionic conductor. The ionic current differs from that in an electronic conductor in that ions are actively involved in the transport of electrons through the solution. The current is a unidirectional flow of electrons through the solution by ionic conductance.

One electrode serves as a source of electrons (cathode) and another serves as a sink for uptake of electrons (anode). At the electron source, ions are electronated or reduced. At the electron sink, ions are de-electronated or oxidized. As an example, hydrogen ions are electronated and form hydrogen molecules at the electronating electrode. At the electron sink electrode oxygen is formed by de-electronation of water. Other ions can undergo the same process.

Many of the products of electrolysis are detrimental to the electroporation process. They interfere with the electrode-ionic conductor interface and they can be toxic to cells. In addition, metals from the electrode can be introduced into the solution by electrolysis or corrosion.

Bipolar pulses reverse the polarity of the electrodes. This causes the electronation electrode to become the de-electronation electrode and the de electronation electrode to become the electronation electrode. This reversal causes a reversal of electrochemistry effects and thus reduces the negative effects of unipolar pulses.

From a study of the prior art, and from the present inventors discoveries, a number of conclusions have been derived relating to electrical pulses, electroporation, deleterious electrode effects, and cell uptake of treating agents. First, although unipolar pulses can provide an electroporation environment in which good macromolecule electrophoresis and good levels of cell uptake of treating agents are obtained, deleterious electrode effects are a problem. Second, and in contradistinction with the first, although bipolar pulses can provide an electroporation environment in which deleterious electrode effects are not a problem, poor macromolecule electrophoresis and poor levels of cell uptake of treating agents are obtained. In this respect, it would be desirable if an electroporation system were provided in which the benefits of using unipolar pulses were obtained without incurring the disadvantages of unipolar pulses, and in which the benefits of using bipolar pulses were obtained without incurring the disadvantages of the bipolar pulses.

More specifically, it would be desirable to provide a system of treating biological materials with electrical fields and treating agents which employs unipolar pulses but which has minimal deleterious electrolytic effects at the electrodes.

Also, it would be desirable to provide a system of treating biological materials with electrical fields and treating agents which employs unipolar pulses and retains good electrophoresis properties for good cell uptake of treating agents.

Also, it would be desirable to provide a system of treating biological materials with electrical fields and treating agents which employs unipolar pulses but which also employs electrode polarity reversal.

Also, it would be desirable to provide a system of treating biological materials with electrical fields and treating agents which employs electrode polarity reversal without employing bipolar pulses.

Thus, while the foregoing body of prior art indicates it to be well known to use electroporation and electrophoresis for driving treating agents into cells, the prior art described above does not teach or suggest a system for delivering therapeutic treating agents into living biological cells, especially living mammalian cells, which has the following combination of desirable features: (1) can produce a pulsed electric field over the treatment volume that is uniform and unidirectional; (2) can be scaled to produce the same uniform and unidirectional electric field over larger or smaller treatment volumes with the same applied pulse voltages (3) can produce, without removing the electrode array a second uniform and unidirectional electric field over the treatment volume at 90 degrees or 180 degrees or 270 degrees with respect to the direction of the first electric field;
(4) can produce, without removing the electrode array, a third or fourth uniform and unidirectional electric field over the treatment volume that are 90 degrees or 180 degrees or 270 degrees with respect to the direction of the first electric field;
(5) can minimize adverse electrochemistry activity at the electrodes, without using bipolar electric fields, when pulse widths that are longer than a few hundred microseconds are used;
(6) can reduce heating in the treatment volume by applying the electric field sequentially to adjacent segments of the treatment volume;
(7) provides an electroporation system in which the benefits of using unipolar pulses are obtained without incurring the disadvantages of unipolar pulses;
(8) provides an electroporation in which the benefits of using bipolar pulses are obtained without incurring the disadvantages of the bipolar pulses;
(9) provides a system for treating biological materials with electrical fields and treating agents which employs unipolar pulses but which has minimal deleterious electrolytic effects at the electrodes;
(10) provides a system for treating biological materials with electrical fields and treating agents which employs unipolar pulses and retains good electrophoresis properties for good cell uptake of treating agents,
(11) provides a system for treating biological materials with electrical fields and treating agents which employs unipolar pulses, but which also employs electrode polarity reversal; and
(12) provides a system for treating biological materials with electrical fields and treating agents which employs electrode polarity reversal without employing bipolar pulses.

Document US 6,208, 893 discloses an apparatus for the delivery of therapeutic compounds in vivo or in vitro into living cells including all the features of the preamble of claim 1.

The foregoing desired characteristics are provided by the unique system of the invention for treating biological materials with translating electrical fields and electrode polarity reversal for driving treating agents into the biological materials. More aspects of the present invention as will be made apparent from the following description thereof. Other advantages of the present invention over the prior art also will be rendered evident.

### Disclosure of Invention

It is noted that this application is related to pending U. S. provisional patent application for METHOD OF TREATING BIOLOGICAL MATERIALS WITH TRANSLATING ELECTRICAL FIELDS AND ELECTRODE POLARITY REVERSAL, Serial Number 60/372, 436, Filing Date 16 April 2002. In addition, aspects of the invention have been disclosed in pending U. S. patent application for ELECTRODES COATED WITH TREATING AGENT AND USES THEREOF of King and Walters, Serial Number 09/920, 861, Filing Date 03 August 2001, and in copending PCT International Application Number PCT/US00/00014, filed 12 January 2000, which is based upon copending United States Provisional Application Serial No. 60/117,755, filed 28 January 1999. The PCT International Application Number PCT/US00/00014 was published on 3 August 2000 with PCT International Publication Number WO 00/44438, which is incorporated herein by reference. In addition to currently disclosing some of those aspects of the invention previously disclosed in the above-mentioned PCT and the above-mentioned United States Provisional Application Serial No. 60/117,755, filed 28 January 1999, the present application discloses additional invention aspects.

This application relates to treating biological cells. The biological cells can be in vivo, ex vivo, or in vitro. More specifically, the biological cells can be in epidermal tissue and can be Langerhans cells in the epidermal tissue. Also, the biological cells can be in deep tissues, and can be in tumors in deep tissues.

The principles of the present invention can be stated in a number of ways.

In accordance with one aspect of the present invention, a system for treating material with a treating agent is provided using pulsed electrical fields provided by a waveform generator. The system includes the steps of:
obtaining an electrode assembly which includes three or more parallel rows of individual electrodes,
establishing electrically conductive pathways between the electrodes and the waveform generator, and
applying successive electric fields in the form of successive electric field waveforms from the waveform generator to adjacent rows of electrodes, wherein each successive electric field has the same direction, and
wherein polarities of rows of electrodes are reversed successively during the applying of the successive electric fields between adjacent successive rows of electrodes.

In accordance with another aspect of the present invention, a system for treating material with an agent is provided using pulsed electrical fields provided by a waveform generator. The system includes the steps of :
a. obtaining an electrode assembly which includes K rows of electrodes, where K is at least three, wherein each successive row of electrodes is spaced apart from a preceding row of electrodes;
b. establishing electrically conductive pathways between the K rows of electrodes and the waveform generator, and
c. providing successive electric fields in the form of successive electric field waveforms from the waveform generator to the K rows of electrodes, wherein each electric field has the same direction,
   (a) such that an Lth electric field is applied between a selected Lth row of electrodes and an (L+1)th row of electrodes among the K rows of electrodes, wherein L+2 is less than or equal to K, wherein the Lth row of electrodes has a first polarity, and the (L+1)th row of electrodes has a second polarity, and
   (b) such that, subsequently, an (L+1)th electric field is applied between the (L+1) th row of electrodes and an (L+2) th row of electrodes wherein the (L+1)th row of electrodes has the first polarity, and the (L+2) th row of electrodes has the second polarity, and
d. repeating step c. as many times as desired with as many selections of L as desired, such that L+2 is less than or equal to K.

Each of the K rows of electrodes can include at least three individual electrodes.

The electric field waveforms can be pulsed electric field waveforms.

The electric field waveforms can be unipolar electric field waveforms.

The pulsed electric field waveforms can be from rectangular pulses.

The pulsed electric field waveforms can be from electrical pulses which are in a sequence of at least three non-sinusoidal electrical pulses, has field strengths equal to or greater than 100 V/cm, to the material, wherein the sequence of at least three non-sinusoidal electrical pulses has one, two, or three of the following characteristics (1) at least two of the at least three pulses differ from each other in pulse amplitude, (2) at least two of the at least three pulses differ from each other in pulse width, and (3) a first pulse interval for a first set of two of the at least three pulses is different from a second pulse interval for a second set of two of the at least three pulses.

The first polarity can be positive, and the second polarity can be negative. Alternatively, the first polarity can be negative, and the second polarity can be positive.

Successive electric fields can be applied unidirectionally from the first and second rows of electrodes to the Kth row of electrodes. Then, successive electrical fields can be applied unidirectionally from the Kth row of electrodes and (K-1)th row of electrodes to the first row of electrodes, which is in reverse direction.

The material treated can be biological material. The biological material can be cellular material. The cellular material can be skin cells, tissue, deep organ tissue, muscle tissue, and mammalian cells, among others.

The treating agent can include molecules of electrode releasable tissue treating agent on the electrodes, which are released from the electrodes by applying electrophoretic pulses to the electrodes. The molecules of the electrode releasable tissue treating agent can be released from the electrodes by contacting the electrodes with a solvent.

In accordance with another aspect of the present invention, a system for immunotherapy is provided which includes the steps of:
a. obtaining an electrode assembly which includes K rows of electrodes, where K is at least three, wherein each successive row of electrodes is spaced apart from a preceding row of electrodes, wherein each electrode is statically-coated with an immuno-stimulating material,
b. establishing electrically conductive pathways between the K rows of electrodes and a waveform generator,
c. inserting the statically-coated electrodes into a tissue to be treated,
d. releasing the immuno-stimulating material from the electrodes,
e. providing successive electric fields in the form of successive electric field waveforms from the waveform generator to the K rows of electrodes, such that the released immuno-stimulating material is driven into cells in the tissue, wherein each electric field has the same direction,
   (a) such that an Lth electric field is applied between a selected Lth row of electrodes and an (L+1)th row of electrodes among the K rows of electrodes, wherein L+2 is less than or equal to K. wherein the Lth row of electrodes has a first polarity, and the (L+1)th row of electrodes has a second polarity, and
   (b) such that, subsequently, an (L+1) th electric field is applied between the (L+1)th row of electrodes and an (L+2)th row of electrodes, wherein the (L+1)th row of electrodes has the first polarity, and the (L+2) th row of electrodes has the second polarity, and
f. repeating step e. as many times as desired with as many selections of L as desired, such that L+2 is less than or equal to K.

The molecules in the static coating can be a solid phase, a gel, and macromolecules such as a polynucleotide vaccine, a solid phase polynucleotide vaccine, a DNA vaccine, a solid phase DNA vaccine, an RNA vaccine, a solid phase RNA vaccine, a protein-based vaccine, a solid phase protein-based vaccine, an organ treating agent, and a deep tissue tumor treating agent, among others.

The immuno-stimulating material can be released from the electrodes by applying electrophoretic pulses to the electrodes. Alternatively, the immuno-stimulating material can be released from the electrodes by contacting the electrodes with a solvent. The immuno-stimulating material can be released from the electrodes by contacting the electrodes with a solvent which includes body fluids.

The electrode assembly can include a plurality of electrodes arranged in at least three parallel rows of electrodes. The at least three parallel rows of electrodes can include at least three parallel plate electrodes.

The parallel rows of electrodes can include needle electrodes. The needle electrodes can include relatively short needles that penetrate skin only. The needle electrodes can include relatively long needles that penetrate tissues bellow the skin. The parallel rows of electrodes can include pad electrodes.

In accordance with another aspect of the present invention, a system for treating material is provided using pulsed electrical fields provided by a waveform generator. The system includes the steps of :
obtaining an electrode assembly which includes a first electrode, a second electrode spaced apart from the first electrode, and a third electrode spaced apart from the second electrode,
establishing electrically conductive pathways between the electrodes and the waveform generator,
locating the electrodes such that the material to be treated is situated therebetween, and
providing- successive electric fields in a common direction in the form of successive pulse waveforms from the waveform generator applied to the material to be treated in the common direction, such that a first electric field is applied between the first electrode and the second electrode, wherein the first electrode has a first polarity, and the second electrode has a second polarity, and such that a second electric field is applied between the second electrode and the third electrode,
wherein the second electrode has the first polarity, and the third electrode has the second polarity, wherein the first electric field and the second electric field are in a common straight line direction.

The electrode assembly can further include a fourth electrode which is spaced apart from the third electrode, and which is located in the material to be treated, further providing an additional electric field in the form of an additional pulse waveform from the waveform generator applied to the material to be treated, such that a third electric field is applied between the third electrode and' the fourth electrode. The third electrode has the first polarity, and the fourth electrode has the second polarity. The first, second, and third electric fields are in a common straight line direction.

The electrode assembly can further include a fifth electrode which is spaced apart from the fourth electrode, and which is located in the material to be treated, further providing an additional electric field in the form of an additional pulse waveform from the waveform generator applied to the material to be treated, such that a fourth electric field is applied between the fourth electrode and the fifth electrode, wherein fourth electrode has the first polarity, and the fifth electrode has the second polarity. The first, second, third, and fourth electric fields are in a common straight line direction.

In accordance with another aspect of the present invention, a system for providing pulsed electrical fields provided by a waveform generator includes the steps of:
a. obtaining an electrode assembly which includes K rows of electrodes, where K is at least three, wherein each successive row of electrodes is spaced apart from a preceding row of electrodes,
b. establishing electrically conductive pathways between the K rows of electrodes and the waveform generator, and
c. providing successive electric fields in the form of successive electric field waveforms from the waveform generator to the K rows of electrodes, wherein each electric field has the same direction,
   (a) such that an Lth electric field is applied between a selected Lth row of electrodes and an (L+1)th row of electrodes among the K rows of electrodes; wherein L+2 is less than or equal to K, wherein the Lth row of electrodes has a first polarity, and the (L+1)th row of electrodes has a second polarity, and
   (b) such that, subsequently, an (L+1)th electric field is applied between the (L+1)th row of electrodes and an (L+2)th row of electrodes, wherein the (L+1)th row of electrodes has the first polarity, and the (L+2) th row of electrodes has the second polarity, and
d. repeating step c. as many times as desired with as many selections of L as desired, such that L+2 is less than or equal to K.

In accordance with another aspect of the present invention, a system treating material with a treating agent is provided using pulsed electrical fields provide by a waveform generator includes the steps of:
obtaining an electrode assembly which includes an array of electrodes which includes at least nine individual electrodes arrayed in a matrix of at least three parallel rows of electrodes and at least three parallel columns of electrodes,
establishing electrically conductive pathways between the individual electrodes and the waveform generator,
applying successive electric fields in the form of successive electric field waveforms from the waveform generator to adjacent parallel rows of electrodes, wherein each successive electric field has the same first direction, and wherein polarities of rows of electrodes are reversed successively during the applying of the successive electric fields between adjacent successive rows of electrodes in the first direction, and
applying successive electric fields in the form of successive electric field waveforms from the waveform generator to adjacent parallel columns of electrodes,
wherein each successive electric field has the same second direction, and wherein polarities of columns of electrodes are reversed successively during the applying of the successive electric fields between adjacent successive columns of electrodes in the second direction, wherein the second direction is orthogonal to the first direction.

All individual electrodes in a row of electrodes can be permanently connected together, and each row of electrodes can be connected to the array switch Alternatively, all electrodes can be individually connected to the array switch.

The electrodes can be needle electrodes. The electric field intensities produced by the electrodes can be 200 v/cm or greater. The electric pulse generator can produce one pulse per pair of rows of electrodes addressed by the array switch. The electric pulse generator can produce rectangular pulses from 1 microsecond to 1 second.

In accordance with another aspect of the present invention, an electrode assembly is provided for connection to an array switch which is connected to a pulse generator. The electrode assembly includes an array of electrodes which includes at least nine individual electrodes arrayed in a matrix of at least three parallel rows of electrodes and at least three parallel columns of electrodes, wherein each of the at least nine individual electrodes is connected individually to the array switch.

With respect to the electrode assembly, each individual electrode is selectively connected to either a pulse generator anode, or a pulse generator cathode, or a neutral potential.

In accordance with another aspect of the present invention, a combination of an electrode assembly and an array switch is provided which is connected to a pulse generator. The combination includes an electrode assembly which includes an array of electrodes which includes at least nine individual electrodes arrayed in a matrix of at least three parallel rows of electrodes and at least three parallel columns of electrodes, and an array switch is connected to the array of electrodes, wherein each of the at least nine individual electrodes is connected individually to the array switch.

Each individual electrode can selectively connected through the array switch to either a pulse generator anode, or a pulse generator cathode, or a neutral potential.

In accordance with another aspect of the present invention, apparatus is provided for the delivery of therapeutic compounds into biological cells. The apparatus includes a waveform generator. An array switch is electrically connected to the waveform generator. An electrode assembly is provided which includes an array of electrodes which includes at least nine individual electrodes arrayed in a matrix of at least three parallel rows of electrodes and at least three parallel columns of electrodes. The array of electrodes is electrically connected to the array switch. Each of the at least nine individual electrodes is connected individually to the array switch.

Each individual electrode can be selectively connected through the array switch to either a waveform generator anode; or a waveform generator cathode, or a neutral potential.

In accordance with another aspect of the present invention, apparatus is provided for the delivery of therapeutic compounds into biological cells in a treatment area. The apparatus includes a waveform generator. An array switch is electrically connected to the waveform generator. An electrode assembly is provided for placement upon the treatment area. The electrode assembly includes an array of electrodes which includes at least nine individual electrodes arrayed in a matrix of at least three parallel rows of electrodes and at least three parallel columns of electrodes. The array of electrodes is electrically connected to the array switch. Each of the at least nine individual electrodes is connected individually to the array switch, wherein each individual electrode is selectively electrically connected through the array switch to either a waveform generator anode, or a waveform generator cathode, or a neutral potential.

Successive electric fields are applied to the treatment area in the form of successive electric field waveforms from the waveform generator to adjacent parallel rows of electrodes, wherein each successive electric field has the same first direction, and wherein polarities of rows of electrodes are reversed successively during the applying of the successive electric fields between adjacent successive rows of electrodes in the first direction.

In addition, successive electric fields are applied to the treatment area in the form of successive electric field waveforms from the waveform generator to adjacent parallel columns of electrodes, wherein each successive electric field has the same second direction, and wherein polarities of columns of electrodes are reversed successively during the applying of the successive electric fields between adjacent successive columns Of electrodes in the second direction. The second direction is orthogonal to the first direction.

The above brief description sets forth rather broadly the more important features of the present invention in order that the detailed description thereof that follows may be better understood, and in order that the present contributions to the art may be better appreciated. There are, of course, additional features of the invention that will be described hereinafter and which will be for the subject matter of the claims appended hereto.

In this respect, before explaining preferred embodiments of the invention in detail, it is understood that the invention is not limited in its application to the details of the construction and to the arrangements of the components set forth in the following description or illustrated in the drawings. The invention is capable of other embodiments and of being practiced and carried out in various ways. Also, it is to be understood, that the phraseology and terminology employed herein are for the purpose of description and should not be regarded as limiting.

As such, those skilled in the art will appreciate, that the conception, upon which disclosure is based, may readily be utilized as a basis for designing other structures, and systems for carrying out the several purposes of the present invention. It is important, therefore, that the claims be regarded as including such equivalent constructions insofar as they do not depart from the scope of the claims.

In view of the above, it is an object of the present invention is to provide a new and improved system for treating biological materials with translating electrical fields and electrode polarity reversal which provides an electroporation system in which the benefits of using unipolar pulses are obtained without incurring the disadvantages of unipolar pulses;

Still another object of the present invention is to provide a new and improved system for treating biological materials with translating electrical fields and electrode polarity reversal that provides an electroporation system in which the benefits of using bipolar pulses are obtained without incurring the disadvantages of the bipolar pulses.

Yet another object of the present invention is to provide a new and improved system for treating biological materials with translating electrical fields and electrode polarity reversal which provides a system for treating biological materials with electrical fields and treating agents which employs unipolar pulses but which has minimal deleterious electrolytic effects at the electrodes.

Even another object of the present invention is to provide a new and improved system for treating biological materials with translating electrical fields and electrode polarity reversal that provides a system for treating biological materials with electrical fields and treating agents which employs unipolar pulses and retains good electrophoresis properties for good cell uptake of treating agents.

Still a further object of the present invention is to provide a new and improved system for treating biological materials with translating electrical fields and electrode polarity reversal which provides a system for treating biological materials with electrical fields and treating agents which employs unipolar pulses, but which also employs electrode polarity reversal.

Yet another object of the present invention is to provide a new and improved system for treating biological materials with translating electrical fields and electrode polarity reversal that provides a system for treating biological materials with electrical fields and treating agents which employs electrode polarity reversal without employing bipolar pulses.

Additional advantages and the specific objects attained by its uses, reference should be had to the accompanying drawings and descriptive matter in which there are illustrated preferred embodiments of the invention.

### Brief Description of Drawings

The invention will be better understood and the above objects as well as objects other than those set forth above will become more apparent after a study of the following detailed description thereof. Such description makes reference to the annexed drawing wherein:
FIG. 1 illustrates a schematic diagram of a system used to produce the unidirectional, uniform electric fields.
FIG. 2 illustrates a schematic diagram of a specific arrangement of polarities for an array of electrodes as determined by a specific arrangement of switches in the array switch.
FIG. 3A schematically illustrates electric fields using two needles per row of electrodes, wherein, in the left side of FIG. 3A, two rows of electrodes are spaced from each other by 4 mm, and wherein, in the right side of FIG. 3A, two rows of electrodes are spaced from each other by 6 mm.
FIG. 3B schematically illustrates electric fields using six needles per row of electrodes, wherein, in the left side of FIG. 3B, two rows of electrodes are spaced from each other by 4 mm, and wherein, in the right side of FIG. 3B, two rows of electrodes are spaced from each other by 6 mm.
FIG. 4A is similar to PIG. 3B, left side, showing the electric field for a row of six anodes diametrically opposite a row of six cathodes.
FIG. 4B illustrates electric fields between a top row of four anodes, a parallel middle row of five cathodes, and a parallel bottom row of four anodes,
wherein electrodes in each row of electrodes are equidistant from the nearest electrodes in the adjacent row or rows of electrodes.

FIGS. 5A-5D schematically illustrate the progressive, unidirectional movement of the electric field vector through sequentially selected rows of electrodes, accompanied by polarity of reversal of the rows of electrodes.

FIG. 6 schematically illustrates the unidirectional movement of a first-direction progressing electric field vector through horizontal rows of electrodes in a first treatment area.

FIG. 7 schematically illustrates the unidirectional movement of a second-direction progressing electric field vector through vertical columns of electrodes in a second treatment area, wherein the second-direction progressing electric field vector is orthogonal to the first-direction progressing electric field vector.

### Modes for Carrying Out the Invention

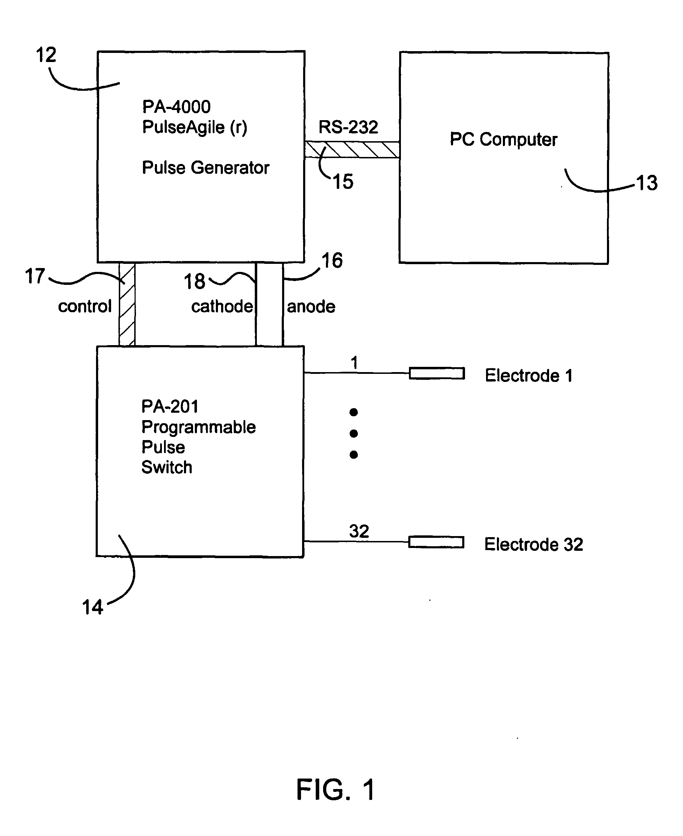

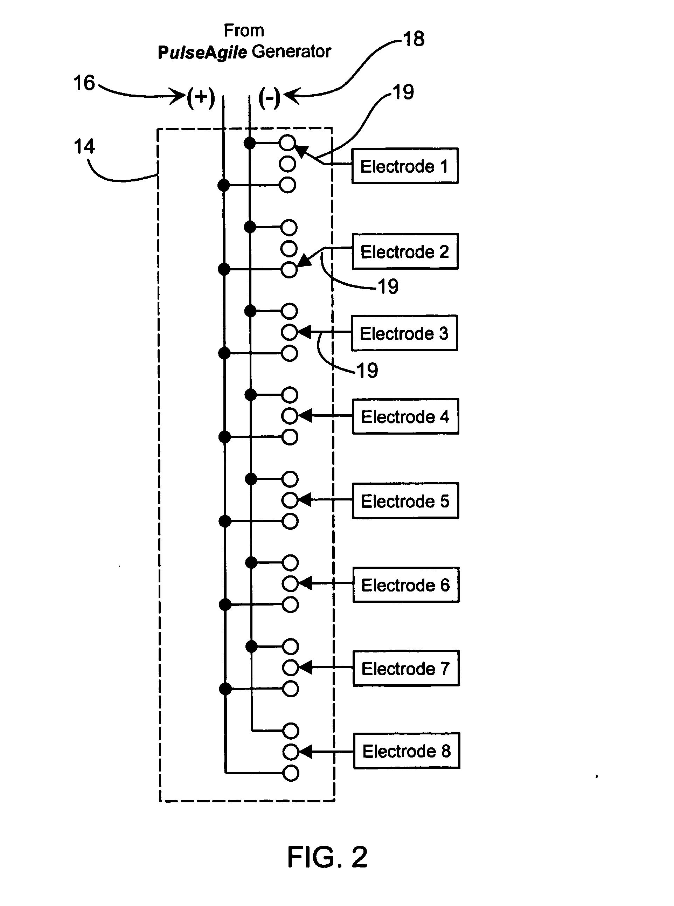

The treatment apparatus uses the system illustrated in FIG. 1. There is a pulse generator 12. A personal computer 13 is interfaced to the pulse generator 12. A RS-232 interface 15 can be used to interface the personal computer 13 to the pulse generator 12. An array switch 14 is connected to the pulse generator anode 16 and pulse generator cathode 18. The array switch 14 is also connected to a neutral or control 17. The array switch 14 is also connected either to each row of electrodes or to each electrode individually in the array of electrodes. One such pulse generator is the Cyto Pulse Sciences, Inc. PA-4000 PulseAgile generator. One such array switch is the Cyto Pulse Sciences, Inc. PA-201 Programmable Pulse Switch. It is noted that the PA-201 Programmable Pulse Switch is capable of being connected to up to thirty-two electrodes or thirty-two rows of electrodes. As shown in FIG. 2. the PA-201 can connect the anode 16 or the cathode 18 of the pulse generator to any row of the electrode array, if the rows are permanently wired together, or to any electrode in the array if the rows of electrodes are not permanently, wired together.

A wide variety of electrodes can be employed. For example, the electrodes can be solid needles, hollow needles, coated needles, uncoated needles, and porous needles.

To more fully appreciate the progressive wave electrode system of the invention, some considerations of electric field in relation to rows of in vivo electrodes will first be discussed.

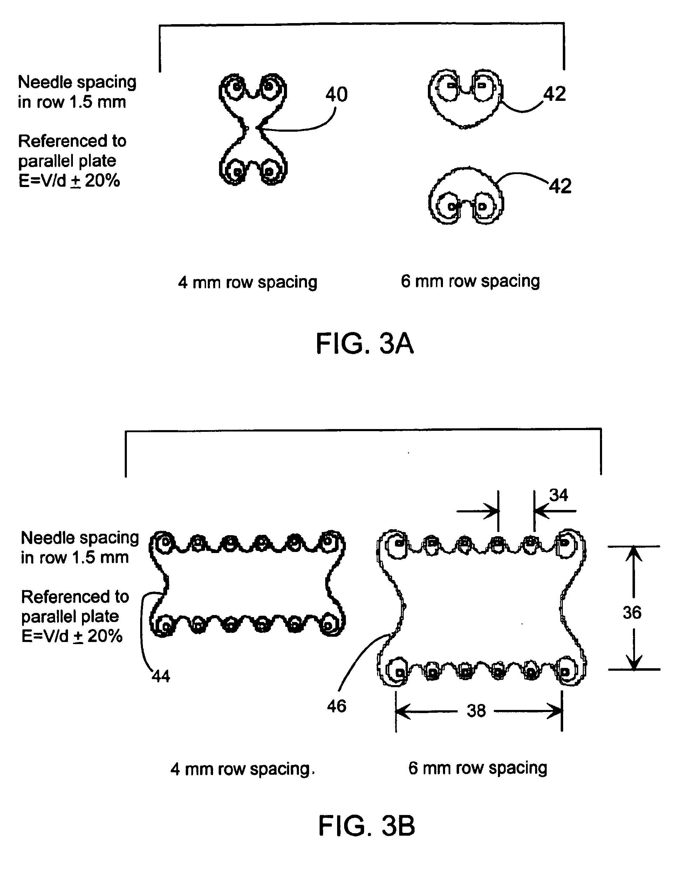

If an electrode array consists of two active parallel rows, and each row consists of two electrodes, and one row is connected to the anode, and one row is connected to the cathode, then the electric field produced is presented in FIG. 3A. The electric field calculation is shown as field lines 40 and 42. Reference is made to a perfect parallel plate with the same outside dimension as the row length and the same spacing as the row spacing. As the spacing between the electrodes in a specific raw increases, or as the spacing between the electrode rows increases or as the needle diameter decreases, the electric field uniformity is degraded.

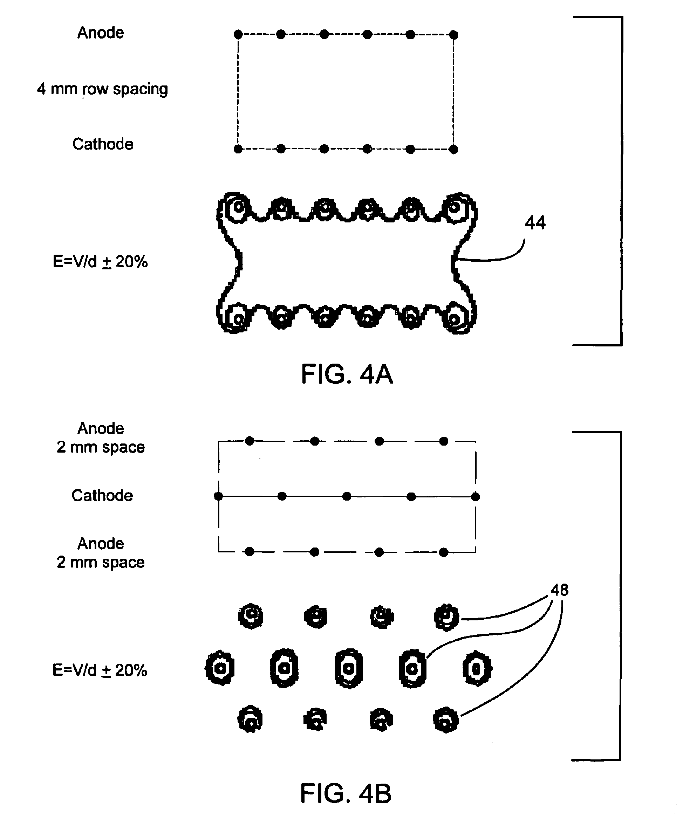

FIG. 3B and FIG. 4A show the electric field calculation for a 6 needle per row electrode. The electric field calculation is shown as field lines 44 and 46. The conclusion of an unpublished internal Cyto Pulse Study dated October 1999 was that a nearly uniform electric field (within 20% of that produced by an equivalent parallel plate) results if:
1. The spacing distance between the electrodes rows 36 is at least 3 times greater than the lateral distance between electrodes in a row 34.
2. The lateral length 38 of an electrode row is at least 2.5 times the spacing distance between the electrodes rows 36.
3. The diameter of the needle electrode is about 0.2 times the spacing distance between the electrodes rows 36.

The electric field produced using just one or two needles per row (such as shown in FIG. 3A) at best can produce only a very narrow uniform electric field.

As shown in FIG. 4B, the equilateral triangle in which one row has one electrode and the second row has two electrodes of opposite polarity has a complex electric field 48 which is not uniform and does not have an electric field vector which is unidirectional (see US Patent 5,873,849). More specifically, FIG. 4B shows the field pattern of a three row equilateral. As shown the equilateral has very limited treat volume coverage and the electric field vectors point in two different directions

To increase the treatment volume the spacing distance between the electrodes rows 36 can be increased. As shown this reduces the uniformity of the electric field at the edges. As the distance increases the uniformity will go to zero. A larger spacing distance between the electrodes rows 36 also requires the increase in voltage to maintain the same electric field intensity in the middle.

Instead of increasing the row spacing to increase the treatment volume, additional rows can be added. If all rows are connected to the pulse generator simultaneously then the polarity of each row must alternate. This causes three problems. The first problem is that the electric field direction changes 180 degrees from one row to the next. The second problem is heating. Adding one row is effectively putting another resistor in parallel thus lowering the internal impedance of the electrode when it is inserted in a conductive media such as living tissue or an aqueous solution. The third problem is that by having more than two row active means, more current from each row is required.

In an unpublished study by Cyto Pulse Sciences dated December 1999 and another dated June 2000 the effect of connecting more than two rows simultaneously was determined. The parameters of the electrodes used are set forth in Table 1 as follows.

**Table 1**

| Parameter | Units | Value December 99 | Value June 00 |
|---|---|---|---|
| Needle radius | mm | 0.082 | 0.15 |
| Needle length | mm | 2.8 | 5.0 |
| Array length | mm | 8.3 | 12.2 |
| Space between needles | mm | 3 | 5 |
| Number of needles | | 9 | 8 |
| Number of rows | | 3 | 8 |

In the December study three rows were used and connected as shown in Table 2 as follows:

**Table 2**

| Run | Row 1 | Row 2 | Row 3 |
|---|---|---|---|
| 1 | + + + + + + | - - - - - - | |
| 2 | | ++++++ | - - - - - - |
| 5 | + + + + + + | - - - - - - | + + + + + + |

The needle array was placed in three homogeneous aqueous solutions and the following resistances were measured, as shown in Table 3.

**Table 3**

| Resistivity' Aqueous solution Ohm-cm | Run 1 Pulse V/I | **Run 2** Pulse V/I | Ran 5 Pulse V/I | Run 5 Ave Run 1 & 2 |
|---|---|---|---|---|
| 63 | 57.5 | 56.8 | 36.4 | 1.57 |
| 120 | 100.0 | 100.0 | 64.9 | 1.54 |
| 240 | 212.8 | 212.8 | 142.9 | 1.49 |

Adding the third row did not reduce the impedance of the electrode by half. This indicated that less current is flowing and thus the electric field is less.

In the June study up to eight electrodes were used in an aqueous solution and in beefsteak. Results of the June study are shown in Table 4. Again the impedance of the array did not decrease as the elementary assumption of adding another similar resistor. Thus as more rows are added the electric field intensity is less than predicted.

**Table 4**

| **Number Of Rows** | **Pulse V/I Aqueous** | **Pulse V/I Beefsteak** |
|---|---|---|
| 2 | 29.4 | 106.7 |
| 3 | 20.5 | 70.6 |
| 4 | 13.7 | 48.0 |
| 5 | 10.7 | 39.8 |
| 6 | 8.90 | 32.9 |
| 7 | 7.36 | 28.1 |
| 8 | 6.09 | 23.7 |

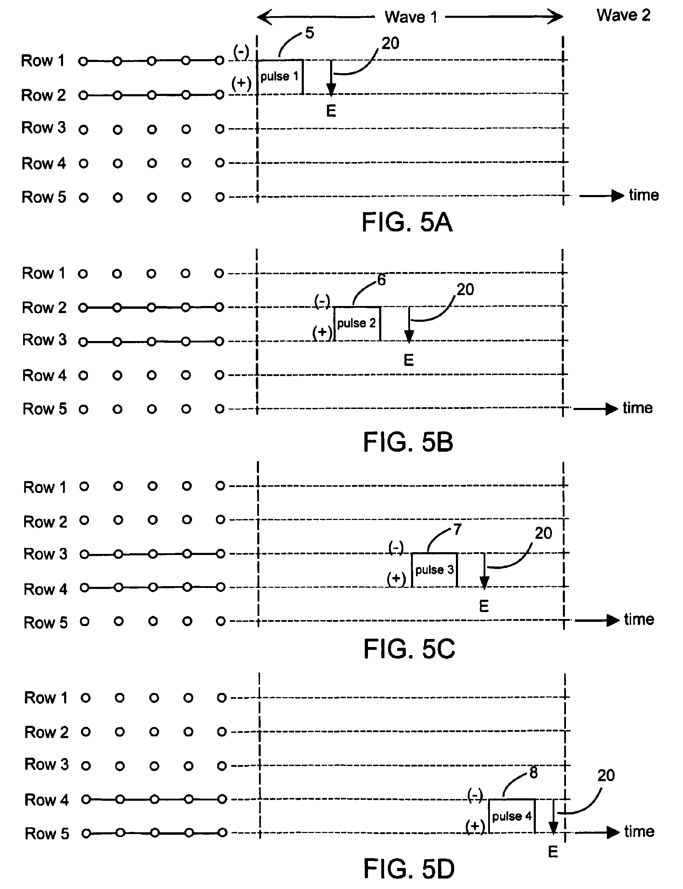

The pulsing configuration for each row of a multi-parallel row electrode with only one pair of rows of electrodes active at a time is shown in FIGS. 5A through 5D.

However, before discussing FIGS. 5A through 5D is detail, attention is first directed to FIG. 2. As shown in FIG. 2, in the array switch 14, each electrode can be connected by a selected switch 19 to either an anode (+) potential, a cathode (-) potential, or a neutral potential. For the specific selections illustrated in FIG. 2, electrode 1 (or electrode row 1) is connected to the cathode potential. Electrode 2 (or electrode row 2) is connected to the anode potential. Electrodes 3-8 (or electrode rows 3-8) are connected to the neutral potential.

Turning to the discussion of FIGS. 5A through 5D, the array switch 14 selections in FIG. 2 correspond to the selections for FIG. 5A.

Subsequently, and not illustrated in FIG. 2, but illustrated in FIG. 5B, for electrode rows 1-5, electrode row 1 is connected to the neutral potential: Electrode row 2 is connected to the cathode potential. Electrode row 3 is connected to the anode potential. Electrode rows 4 and 5 are connected to the neutral potential.

Further, as illustrated in FIG. 5C, electrodes rows 1 and 2 are connected to the neutral potential. Electrode row 3 is connected to the cathode potential. Electrode row 4 is connected to the anode potential. Electrode row 5 is connected to the neutral potential.

Still further, as illustrated in FIG. 5D, electrode rows 1-3 are connected to the neutral potential. Electrode row 4 is connected to the cathode potential. Electrode row 5 is connected to the anode potential.

Clearly, as illustrated in FIGS. 5A through 5D, the electric field vector 20 progressively moves unidirectionally. Moreover, the electric field is uniform at each incremental position in the electric field progression, such as through FIGS 5A through 5D.

Furthermore, polarity reversals occur as the uniform electric field progresses unidirectionally. More specifically, in FIG. 5A, electrode row 2 is connected to the anode potential. In FIG. 5B, electrode row 2 is connected to the cathode potential.

In FIG. 5B, electrode row 3 is connected to the anode potential. In FIG. 5C, electrode row 3 is connected to the cathode potential.

In FIG. 5C, electrode row 4 is connected to the anode potential. In FIG. 5D, electrode row 4 is connected to the cathode potential .

It is understood that for electrode rows 1-5, the respective electrodes in the respective rows can be wired together. Alternatively, the respective electrodes in the respective rows of electrodes can be selected simultaneously by the array switch 14.

In the example in FIGS. 5A through 5D, a five elements by five elements electrode is used. That is, 25 electrodes are arrayed in a matrix having 5 rows and 5 columns. In general, an electrode array used with the present invention can be in a matrix array having K rows and M columns.

The Table 5 below provides the values of various parameters as a function of distance between electrodes assuming the electrode are needles.

**Table 5**

| **Parameter** | **Value** | | | |
|---|---|---|---|---|
| Space Between Needle Centers | 2 mm | 3 mm | 4 mm | 5 mm |
| Coverage | 6x8 mm | 9x12 mm | 12x16 mm | 15x20 mm |
| Time to complete-one wave for Pulse interval of 0.125 seconds | 0.5 seconds | 0.5 seconds | 0.5 seconds | 0.5 seconds |
| Pulse Amplitude for 1200 v/cm | 240 volts | 360 volts | 480 volts | 600 volts |

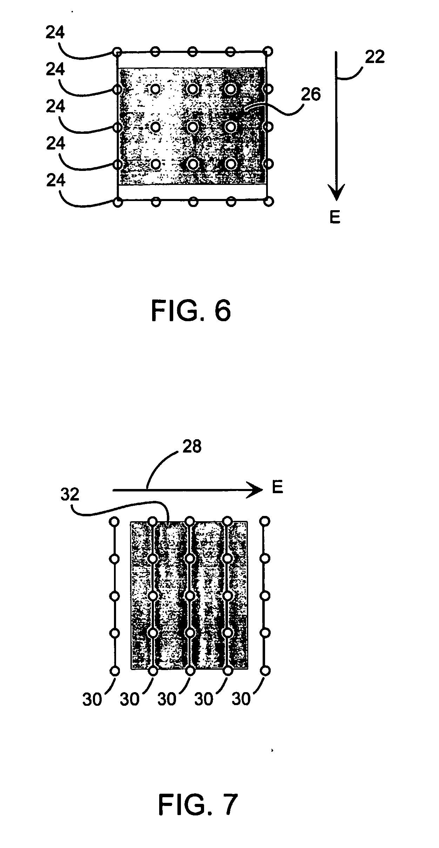

with respect to the "coverage", in FIG. 6 and FIG. 7, the actual area treated is the area inside the 5 X 5 matrix array of electrodes. Also, with respect to FIG. 6 and FIG. 7, each of the twenty-five electrodes in the 5 X 5 matrix array of electrodes is connected to the array switch 14 individually.

More specifically with respect to FIG. 6, the electrodes are selected by the array switch 14 so that groups of horizontal rows of electrodes 24 are selected. In this respect, a first-direction progressing electric field vector 22 is oriented in a vertical direction in FIG. 6. The actual area for ion minimization is first ion minimization area 26 which is less than the area treated by the electric field. In this respect, the first-direction progressing electric field vector 22 is longer than the first ion minimization area 26 in the vertical direction.

More specifically with respect to FIG. 7, the electrodes are selected by the array switch 14 so that groups of vertical columns of electrodes 30 are selected. In this respect, a second-direction progressing electric field vector 28 is oriented in a horizontal direction,in FIG. 7. The actual area for ion minimization is second ion minimization area 32 which is less than the area treated by the electric field. In this respect, the second-direction progressing electric field vector 28 is longer than the second ion minimization area 32 in the horizontal direction.

A treatment regimen can be provided so that a treatment area is treated by (a) a first treatment by a progressive sequence of uniform electric fields advancing through the treatment area in a first direction, accompanied by polarity reversals of electrodes, such as shown in FIG. 6, which is then followed by (b) a second treatment by a progressive sequence of uniform electric fields advancing through the treatment area in a second direction; which is orthogonal to the first direction, accompanied by polarity reversals of electrodes, such as shown in FIG. 7.

It is apparent from the above that the present invention accomplishes all of the objects set forth, which include providing a new and improved system for treating biological materials with translating electrical fields and electrode polarity reversal which may advantageously be used to provide an electroporation system in which the benefits of using unipolar pulses are obtained without incurring the disadvantages of unipolar pulses. With the invention, a system for treating biological materials with translating electrical fields and electrode polarity reversal provides an electroporation method in which the benefits of using bipolar pulses are obtained without incurring the disadvantages of the bipolar pulses. With the invention, a system for treating biological materials with translating electrical fields and electrode polarity reversal provides a system for treating biological materials with electrical fields and treating agents which employs unipolar pulses but which has minimal deleterious electrolytic effects at the electrodes. With the invention, a system for treating biological materials with translating electrical fields and electrode polarity reversal provides a system for treating biological materials with electrical fields and treating agents which employs unipolar pulses and retains good electrophoresis properties for good cell uptake of treating agents, with the invention, a system for treating biological materials with translating electrical fields and electrode polarity reversal is provided which provides a system for treating biological materials with electrical fields and treating agents which employs unipolar pulses, but which also. employs electrode polarity reversal. With the invention a system for treating biological materials with translating electrical fields and electrode polarity reversal is provided which provides a system for treating biological materials with electrical fields and treating agents which employs electrode polarity reversal without employing bipolar pulses.

## Claims

1. An apparatus for the delivery of therapeutic compounds in vivo or in vitro into living cells comprising:
an array of electrodes consisting of three or more parallel rows (1-32) of electrodes with more than three electrodes per row with the electrodes in each row opposing the electrodes in adjacent rows of electrodes,
an electrical pulse voltage generating means (12) with an anode (16) and a cathode (18), and
an array switching means (14) wherein said switching means (14) connects the anode (16) of the pulse voltage generator (12) to a row of electrodes and the cathode (18) of the pulse generator to an adjacent row of opposing electrodes, **characterized in that** said array switching means (14) is operated for successively selecting a succeeding pair (1, 2; 2, 3; 3,4...) of rows of electrodes, such that only one row (2,3,4...) of the next pair (2, 3; 3, 4; 4, 5... ) of rows of electrodes must have been connected during the previous pair connection, and such that the polarity of the common row (2, 3, 4...) of electrodes connected is opposite for the next connected pair of rows of electrodes, and successively connecting the next adjacent rows of electrodes (3, 4; 4, 5;...) in the same manner until all rows of electrodes have been connected to said pulse generator (12).

2. The apparatus of claim 1 wherein all individual electrodes in a row of electrodes are permanently connected and wherein each row of electrodes (1-32) is connected to the array switch (14).

3. The apparatus of claim 1 wherein all electrodes are individually connected to said array switch (14).

4. The apparatus of claim 1 wherein said electrodes are needle electrodes.

5. The apparatus of claim 1 wherein electric field intensities produced by the electrodes are 200 v/cm or greater.

6. The apparatus of claim 1 wherein said electric pulse generator (12) produces one pulse per pair (1, 2; 2, 3; 3, 4...) of rows of electrodes addressed by said array switch.

7. The apparatus of claim 1 wherein said electric pulse generator (12) produces rectangular pulses from 1 microsecond to 1 second.

8. The apparatus of claim 1 wherein the living cells are mammalian cells.

9. The apparatus of claim 1 wherein the living cells are human tissue cells.

10. The apparatus of claim 1 wherein the therapeutic compounds are large molecules.

11. The apparatus of claim 1, wherein said array switching means (14) is operable to produce, without removing the electrode array, a second uniform and unidirectional electric field over the treatment volume at 90 degrees or 180 degrees or 270 degrees with respect to the direction of the first electric field applied by connecting the anode (16) and cathode (18) of the pulse generator (12) to said rows of electrodes (1-32).

## Patentansprüche

1. Vorrichtung zur Abgabe von therapeutischen Verbindungen in vivo oder in vitro in lebende Zellen mit:
einem Elektrodenarray, das aus drei oder mehr parallelen Elektrodenreihen (1-32) mit mehr als drei Elektroden pro Reihe besteht, wobei die Elektroden in jeder Reihe den Elektroden in benachbarten Elektrodenreihen gegenüberliegen,
einem Mittel (12) zum Erzeugen elektrischer Spannungspulse mit einer Anode (16) und einer Kathode (18), und
einem Arrayschaltungsmittel (14), wobei das Schaltungsmittel (14) die Anode (16) des Spannungspulsgenerators (12) mit einer Elektrodenreihe und die Kathode (18) des Pulsgenerators mit einer benachbarten Reihe gegenüberliegender Elektroden verbindet,
**dadurch gekennzeichnet, dass** das Arrayschaltungsmittel (14) betrieben wird zum sukzessiven Auswählen eines Folgepaares (1, 2; 2, 3; 3, 4 ...) von Elektrodenreihen, so dass nur eine Reihe (2, 3, 4 ...) des nächsten Paares (2, 3; 3, 4; 4, 5 ...) von Elektrodenreihen während der vorausgehenden Paarverbindung verbunden sein muss, und so dass die Polarität der gemeinsamen Reihe (2, 3, 4 ...) verbundener Elektroden entgegengesetzt ist für das nächste verbundene Paar von Elektrodenreihen, und wobei die nächsten benachbarten Elektrodenreihen (3, 4; 4, 5; ...) nacheinander in der gleichen Weise verbunden werden, bis alle Elektrodenreichen mit dem Pulsgenerator (12) verbunden sind.

2. Vorrichtung nach Anspruch 1, wobei alle individuellen Elektroden in einer Elektrodenreihe permanent verbunden sind, und wobei jede Elektrodenreihe (1-32) mit dem Arrayschalter (14) verbunden ist.

3. Vorrichtung nach Anspruch 1, wobei alle Elektroden individuell mit dem Arrayschalter (14) verbunden sind.

4. Vorrichtung nach Anspruch 1, wobei die Elektroden Nadelelektroden sind.

5. Vorrichtung nach Anspruch 1, wobei elektrische Feldstärken, die durch die Elektroden erzeugt werden, 200 V/m oder größer sind.

6. Vorrichtung nach Anspruch 1, wobei der elektrische Pulsgenerator (12) einen Puls pro Paar (1, 2; 2, 3; 3, 4 ...) von Elektrodenreihen erzeugt, die von dem Arrayschalter adressiert sind.

7. Vorrichtung nach Anspruch 1, wobei der elektrische Pulsgenerator (12) rechteckige Pulse von 1 Mikrosekunde bis zu 1 Sekunde erzeugt.

8. Vorrichtung nach Anspruch 1, wobei die lebenden Zellen Säugetierzellen sind.

9. Vorrichtung nach Anspruch 1, wobei die lebenden Zellen menschliche Gewebezellen sind.

10. Vorrichtung nach Anspruch 1, wobei die therapeutischen Verbindungen große Moleküle sind.

11. Vorrichtung nach Anspruch 1, wobei das Arrayschaltungsmittel (14) betriebsfähig ist, ohne Entfernen des Elektrodenarrays ein zweites uniformes und unidirektionales elektrisches Feld über dem Behandlungsvolumen in einem Winkel von 90 Grad oder 180 Grad oder 270 Grad in Bezug auf die Richtung des ersten elektrisches Feldes zu erzeugen, das durch Verbinden der Anode (16) und der Kathode (18) des Pulsgenerators (12) mit den Elektrodenreihen (1-32) angelegt wird.

## Revendications

1. Dispositif de fourniture de composés thérapeutiques in vivo ou in vitro dans des cellules vivantes, comprenant :
un ensemble d'électrodes composé de trois rangées parallèles (1-32) ou plus d'électrodes comprenant plus de trois électrodes par rangée dont les électrodes dans chaque rangée sont opposées aux électrodes dans les rangées d'électrodes adjacentes,
un moyen de génération de tension à impulsion électrique (12) muni d'une anode (16) et d'une cathode (18), et
un moyen de commutation d'ensemble (14), dans lequel ledit moyen de commutation (14) relie l'anode (16) du générateur de tension d'impulsion (12) à une rangée d'électrodes et la cathode (18) du générateur d'impulsion à une rangée adjacente d'électrodes opposées, **caractérisé en ce que** ledit moyen de commutation d'ensemble (14) est utilisé pour sélectionner successivement une paire suivante (1, 2; 2, 3 ; 3, 4, ...) de rangées d'électrodes, de telle sorte que seulement une rangée (2, 3, 4, ...) de la paire suivante (2, 3 ; 3, 4 ; 4, 5 ; ...) de rangées d'électrodes doit avoir été reliée pendant la connexion de la paire précédente, et de telle sorte que la polarité de la rangée commune (2, 3, 4, ...) d'électrodes reliées soit inverse à la paire reliée suivante de rangées d'électrodes, et reliant successivement les rangées d'électrodes (3, 4 ; 4, 5 ; ...) adjacentes suivantes de la même manière jusqu'à ce que toutes les rangées d'électrodes aient été reliées audit générateur d'impulsion (12).

2. Dispositif selon la revendication 1, dans lequel toutes les électrodes individuelles dans une rangée d'électrodes sont reliées en permanence et dans lequel chaque rangée d'électrodes (1-32) est reliée au commutateur d'ensemble (14).

3. Dispositif selon la revendication 1, dans lequel toutes les électrodes sont reliées individuellement audit commutateur d'ensemble (14).

4. Dispositif selon la revendication 1, dans lequel lesdites électrodes sont des électrodes à aiguille.

5. Dispositif selon la revendication 1, dans lequel les intensités du champ électrique produites par les électrodes sont de 200 V/cm ou plus.

6. Dispositif selon la revendication 1, dans lequel ledit générateur d'impulsion électrique (12) produit une impulsion par paire (1, 2 ; 2, 3; 3, 4 ; ...) de rangées d'électrodes adressées par ledit commutateur d'ensemble.

7. Dispositif selon la revendication 1, dans lequel ledit générateur d'impulsion électrique (12) produit des impulsions rectangulaires de 1 microseconde à 1 seconde.

8. Dispositif selon la revendication 1, dans lequel les cellules vivantes sont des cellules mammaires.

9. Dispositif selon la revendication 1, dans lequel les cellules vivantes sont des cellules de tissus humains.

10. Dispositif selon la revendication 1, dans lequel les composés thérapeutiques sont de grandes molécules.

11. Dispositif selon la revendication 1, dans lequel ledit moyen de commutation d'ensemble (14) est utilisable pour produire, sans retirer l'ensemble d'électrodes, un second champ électrique uniforme et unidirectionnel sur le volume de traitement à 90 degrés ou 180 degrés ou 270 degrés par rapport à la direction du premier champ électrique appliqué en reliant l'anode (16) et la cathode (18) du générateur d'impulsions (12) auxdites rangées d'électrodes (1-32).
